# EUROPEAN PATENT APPLICATION

(11) **EP 4 020 483 A1**
(43) Date of publication of application: **29.06.2022**
(21) Application number: 21202111.7
(22) Date of filing: 12.10.2021
(51) Int. Cl.: G16B 15/30, C07K 7/00, G16C 20/50

(54) **STABLE STRUCTURE SEARCH METHOD, STABLE STRUCTURE SEARCH PROGRAM, AND STABLE STRUCTURE SEARCH DEVICE**

(30) Priority: 28.12.2020 JP 2020218127
(71) Applicant: FUJITSU LIMITED, Kawasaki-shi, Kanagawa 211-8588 (JP)
(72) Inventor: Tanida, Yoshiaki, Kawasaki-shi, Kanagawa, 211-8588 (JP)
(74) Representative: Hoffmann Eitle

(57) **Abstract**

A stable structure search method for a computer to execute a process includes determining an initial structure of a trans-type cyclic peptide so that all of dihedral angles between a carbon atom and a nitrogen atom that form a peptide bond in the trans-type cyclic peptide are from 150° to 210°; and perform a first molecular simulation of a structure of the trans-type cyclic peptide by using the initial structure.

## Description

### FIELD

The embodiments discussed herein are related to a stable structure search method, a stable structure search program, and a stable structure search device.

### BACKGROUND

In recent years, cyclic peptides that can be orally administered, have excellent in vivo stability, and are expected to have high binding activity are expected as promising candidates for medium-molecular-weight drugs. More specifically, the cyclic peptides are promising as candidates for new drug discovery because its entropy is greatly reduced by cyclization, so that its binding activity can be increased.

To narrow down (screen) the cyclic peptides favorable as a medium-molecular-weight drug in consideration of physical properties such as binding activity and membrane permeability, it is important to specify a stable structure of the cyclic peptides in a solvent.

However, since the structure of the cyclic peptides has a large fluctuation (a large degree of freedom) or the like, it may be difficult to determine the structure by an experimental method such as X-ray crystal structure analysis or NMR. Therefore, when determining the structure of the cyclic peptides, it is important to search for the stable structure by a computational method such as molecular dynamics simulation.

As a technique for searching for a stable structure of cyclic peptides by a computational method, for example, a technique for searching for a space by combining various combinations of structures on the basis of quantitative parameters in each combination has been proposed. Furthermore, as a technique for stabilizing cyclic peptides by a computational method, for example, a technique for calculating a van der Waals force after inserting an unnatural amino acid to obtain a solvent accessible surface area-based energy has been proposed.

Here, since the cyclic peptides have a high degree of freedom in structure, as described above, there may be a large number of structures having close minimum values (valleys of similar depth) on the free energy curved surface. Moreover, in the cyclic peptides, there may be an energy barrier (high peak or barrier) that is not able to be easily crossed at room temperature.

Therefore, when searching for the stable structure of the cyclic peptides, simply performing a normal molecular dynamics simulation at room temperature will cause the structure of the cyclic peptides to drop in a certain minimum value (a valley of a local solution), and the stable structure may not be able to be searched in a wide range of structural topological space.

An example of a method of suppressing the drop (trap) of the structure into the valley of a local solution and sampling the structure in the structural topological space in the widest possible range includes a molecular dynamics simulation using an extended ensemble method.

However, in such an existing technique, it may be difficult to cross the energy barrier of the free energy curved surface when sampling the structure, depending on an initial structure of the cyclic peptides of interest. That is, in such an existing technique, when sampling the structure of the cyclic peptides by the molecular dynamics simulation or the like, the structure of the cyclic peptides as the initial structure may affect a sampling result, and the sampled structure may be biased.

Japanese National Publication of International Patent Application No. 2003-524831 and Japanese National Publication of International Patent Application No. 2005-512161 are disclosed as related art.

### SUMMARY

### [TECHNICAL PROBLEM]

In one aspect, an objective of the present case is to provide a stable structure search method for a cyclic peptide, a stable structure search program for a cyclic peptide, and a stable structure search device for a cyclic peptide capable of appropriately sampling a structure of the cyclic peptide and capable of appropriately searching for a stable structure for the cyclic peptide.

### [SOLUTION TO PROBLEM]

According to an aspect of the embodiments, a stable structure search method for a computer to execute a process includes determining an initial structure of a trans-type cyclic peptide so that all of dihedral angles between a carbon atom and a nitrogen atom that form a peptide bond in the trans-type cyclic peptide are from 150° to 210°; and perform a first molecular simulation of a structure of the trans-type cyclic peptide by using the initial structure.

### [ADVANTAGEOUS EFFECTS OF INVENTION]

In one aspect, the present case can provide a stable structure search method for a cyclic peptide, a stable structure search program for a cyclic peptide, and a stable structure search device for a cyclic peptide capable of appropriately sampling a structure of the cyclic peptide and capable of appropriately searching for a stable structure for the cyclic peptide.

### BRIEF DESCRIPTION OF DRAWINGS

FIG. 1 is a graph illustrating an example of a relationship between free energy and a structural topological space in a cyclic peptide.
FIG. 2 is a graph illustrating an example of a relationship between an initial structure of molecular dynamics simulation using an extended ensemble method and a sampled structure when sampling a structure of the cyclic peptide by the molecular dynamics simulation.
FIG. 3 is a diagram illustrating an example of a relationship between the initial structure of the molecular dynamics simulation and a path for crossing an energy barrier of a free energy curved surface when sampling the structure of the cyclic peptide.
FIG. 4 is a diagram illustrating a hardware configuration example of a stable structure search device for a cyclic peptide disclosed in the present case.
FIG. 5 is a diagram illustrating another hardware configuration example of the stable structure search device for a cyclic peptide disclosed in the present case.
FIG. 6 is a diagram illustrating a functional configuration example of the stable structure search device for a cyclic peptide disclosed in the present case.
FIG. 7 is a diagram illustrating a flowchart illustrating an example of a flow when searching for a stable structure of a cyclic peptide using the technique disclosed in the present case.
FIG. 8 is a diagram illustrating a flowchart illustrating another example of the flow when searching for a stable structure of a cyclic peptide using the technique disclosed in the present case.
FIG. 9 is a diagram illustrating a flowchart illustrating another example of the flow when searching for a stable structure of a cyclic peptide using the technique disclosed in the present case.
FIG. 10 is a diagram illustrating an example of the structure of the cyclic peptide (PDB ID: 6AXI) targeted in the present example.
FIG. 11 is a diagram illustrating an example of the stable structure specified in an experiment at a site where two prolines are consecutively bound in the cyclic peptide (PDB ID: 6AXI) targeted in the present example.
FIG. 12 is a graph illustrating an example of a frequency distribution of a dihedral angle ω of the first proline (Pro1) in a case of selecting, as an initial structure, a structure in which the first proline (Pro1) is a cis type and the second proline (Pro2) is a trans type at a site where the two prolines are consecutively bound in the cyclic peptide targeted in the present example;
FIG. 13 is a graph illustrating an example of the frequency distribution of the dihedral angle ω of the first proline (Pro1) in a case of selecting, as the initial structure, a structure in which the first proline (Pro1) is a cis type and the second proline (Pro2) is also a cis type at a site where the two prolines are consecutively bound in the cyclic peptide targeted in the present example; and
FIG. 14 is a graph illustrating an example of the frequency distribution of the dihedral angle ω of the first proline (Pro1) in a case of selecting, as the initial structure, a structure in which the first proline (Pro1) is a trans type and the second proline (Pro2) is also a trans type at a site where the two prolines are consecutively bound in the cyclic peptide targeted in the present example.

### DESCRIPTION OF EMBODIMENTS

### (Cyclic Peptide Stable Structure Search Method)

The technique disclosed in the present case is based on the knowledge of the present inventor that, in an existing technique, when sampling a structure of a cyclic peptide, the structure of a cyclic peptide as an initial structure may affect a sampling result, and the sampled structure may be biased. Therefore, before describing details of the technique disclosed in the present case, problems and the like of the known technique will be described.

First, as described above, since the cyclic peptide has a high degree of freedom in structure, there may be a large number of structures having close minimum values (valleys of similar depth) on a free energy curved surface. Moreover, in the cyclic peptide, there may be an energy barrier (high peak or barrier) that is not able to be easily crossed at room temperature.

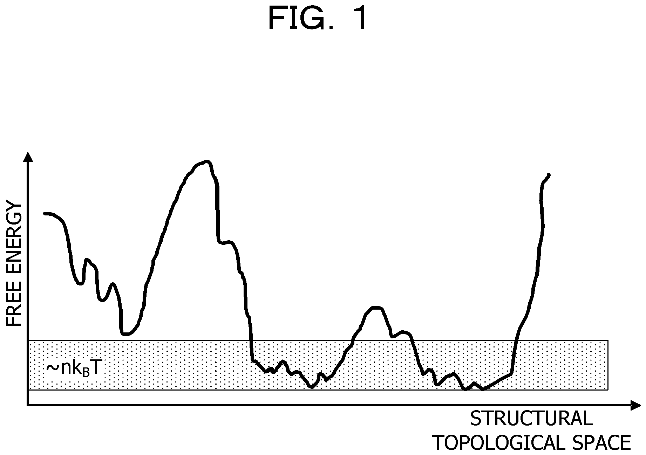

FIG. 1 illustrates an example of a relationship between free energy and a structural topological space (free energy curved surface and free energy profile) in a cyclic peptide. In FIG. 1, the vertical axis represents the free energy and the horizontal axis represents the structural topological space.

As illustrated in FIG. 1, the free energy curved surface of the cyclic peptide may have a large number of minimum values (many valleys of similar depth) having close free energy values. Moreover, as illustrated in FIG. 1, the free energy curved surface of the cyclic peptide may have an energy barrier that is not able to be easily crossed by the degree of structural fluctuation at room temperature (the region indicated by "nk_{B}T" in FIG. 1). Note that, in "nk_{B}T" illustrated in FIG. 1, "n" means the number of atoms, "k_{B}" means the Boltzmann constant, and "T" means the temperature.

As described above, the free energy curved surface of the cyclic peptide has many valleys of similar depth and the energy barrier that is not able to be easily crossed at room temperature. Therefore, when searching for a stable structure of the cyclic peptide, the structure of the cyclic peptide may drop (trap) into a certain minimum value by simply performing a normal molecular simulation at room temperature.

An example of the method of suppressing the drop of the structure into the valley of a local solution and sampling the structure in the structural topological space in the widest possible range includes the molecular dynamics simulation using an extended ensemble method, as described above. In the extended ensemble method, for example, by adding an artificial potential to implement a random walk in the energy space, the drop of the structure into the valley of a local solution can be suppressed.

Here, sampling of the structure of the cyclic peptide by the molecular dynamics simulation using the extended ensemble method can be performed by, for example, the following procedure.

(1) Prepare the initial structure of the cyclic peptide, and determine parameters of a potential function (molecular force field) including atomic charge, van der Waals force, or the like used in the molecular dynamics simulation on the basis of the initial structure (create a model of the cyclic peptide).

(2) Execute the molecular dynamics simulation using the extended ensemble method to suppress the drop of the structure of the cyclic peptide into the valley of a local solution, and sample the structure of the cyclic peptide.

However, in such an existing technique, as described above, it may be difficult to cross the energy barrier of the free energy curved surface when sampling the structure, depending on an initial structure of the cyclic peptide of interest.

Hereinafter, the relationship between the structure of the cyclic peptide and the energy barrier of the free energy curved surface will be more specifically described.

Regarding the structure of the cyclic peptide, a binding state of a main chain (backbone) in the cyclic peptide can be represented by, for example, a dihedral angle in the main chain of a molecule. The dihedral angle of the main chain in the cyclic peptide means an angle between two planes formed by four consecutive atoms with a bond in the main chain of the cyclic peptide.

In the main chain of the cyclic peptide, the following three types of dihedral angles exist for each amino acid residue.
- Dihedral angle ϕ between Cα (asymmetric carbon atom) and N (nitrogen atom)
- Dihedral angle Ψ between Cα (asymmetric carbon atom) and C (carbon atom of a carbonyl group)
- Dihedral angle ω between C (carbon atom of a carbonyl group) and N (nitrogen atom)

In the above dihedral angle, the dihedral angle ϕ and the dihedral angle Ψ can freely rotate to some extent, but the dihedral angle ω is limited to an angle of either vicinity of 0° (cis type) or the vicinity of 180° (trans type). The dihedral angle ω is limited to the vicinity of 0° or the vicinity of 180° because the bond between the carbon atom (carbon atom of a carbonyl group) and the nitrogen atom forming the peptide bond at the dihedral angle ω has a double-bond character.

In this way, the dihedral angle ω is divided into two types of states with significantly different angles: either the vicinity of 0° (cis type) or the vicinity of 180° (trans type), and thus the energy barrier that separates these states is high. More specifically, at the dihedral angle ω of the cyclic peptide, the energy barrier separating the cis type and the trans type has a large value of about 20 kcal/mol.

Therefore, it may be difficult to cross the energy barrier of the free energy curved surface even using the extended ensemble method, depending on the initial structure of the cyclic peptide of interest, particularly, the value of the dihedral angle ω between a carbon atom and a nitrogen atom forming the peptide bond in the initial structure. In other words, there are some cases where the type of dihedral angle ω (cis type or trans type) or the like in the cyclic peptide as the initial structure affects the sampling result, and the sampled structure is biased (an artificially incorrect structure is generated).

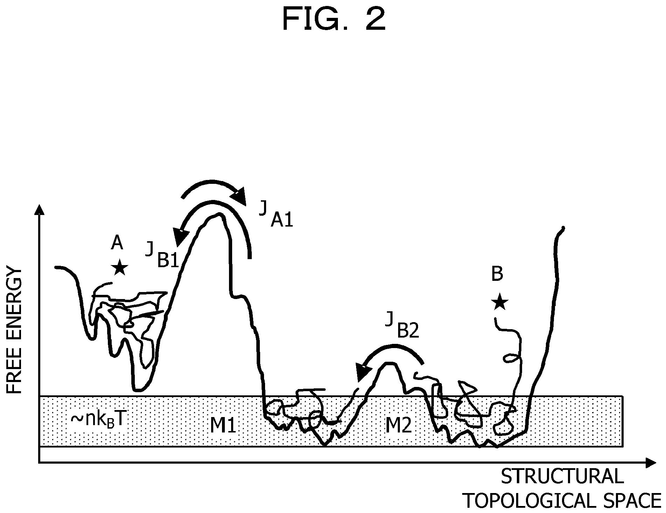

FIG. 2 illustrates an example of a relationship between an initial structure of molecular dynamics simulation using an extended ensemble method and a sampled structure when sampling a structure of the cyclic peptide by the molecular dynamics simulation. In FIG. 2, the vertical axis represents the free energy and the horizontal axis represents the structural topological space. FIG. 2 illustrates sampled structures (structure search range) in a model A having parameters determined on the basis of an initial structure A and a model B having parameters determined on the basis of an initial structure B as an example.

First, in the model A based on the initial structure A, when sampling the structure by the extended ensemble method, a potential barrier M1 is crossed and the valley between potential barriers M1 and M2 is reached, as illustrated by the arrow J_{A1}. However, in the model A based on the initial structure A, the energy barrier M2, which is lower than the potential barrier M1, is not able to be crossed due to the influence of the parameters in the model A, and the structure can be sampled only in a limited structural topological space. Therefore, in the example illustrated in FIG. 2, when the structure is sampled using the initial structure A, appropriate sampling is not able to be performed, and an artificially erroneous structure is generated.

Meanwhile, in the model B based on the initial structure B, when sampling the structure by the extended ensemble method, the potential barrier M2 is crossed and the valley between the potential barriers M1 and M2 is reached, as illustrated by the arrow J_{B2}. Moreover, in the model B based on the initial structure B, the potential barrier M1 can also be crossed, as illustrated by the arrow J_{B1}, and the structure can be sampled in a wide structural topological space. Therefore, in the example illustrated in FIG. 2, when sampling the structure using the initial structure B, appropriate sampling can be performed to include the most stable structure, and the stable structure can be appropriately used in a wide search range.

Note that, in FIG. 2, for convenience of description, the free energy of the model A based on the initial structure A and the model B based on the initial structure B is expressed using a two-dimensional curve. However, the free energy of these models is actually a multidimensional curved surface. Furthermore, the free energy in the model A and the model B is expressed using the same curve, but the curves (curved surfaces) themselves of the free energy are actually different between the model A and the model B because the parameters such as atomic charge and van der Waals force are different.

Therefore, the model A based on the initial structure A and the model B based on the initial structure B actually have different multidimensional free energy curved surfaces themselves, so that paths for crossing the energy barriers M1 and M2 are different. That is, the model A based on the initial structure A and the model B based on the initial structure B differ in energy needed to cross the energy barriers M1 and M2 on the multidimensional free energy curved surfaces. Therefore, whether the energy barriers M1 and M2 can be crossed depends on which of the initial structure A or the initial structure B is used as the initial structure, so that the sampling range of the structure depends on the initial structure.

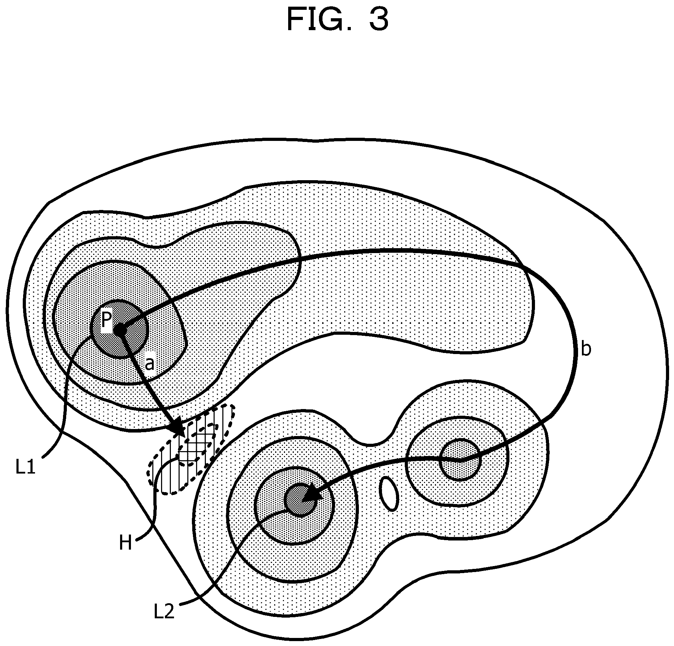

Here, a relationship between the initial structure of the molecular dynamics simulation and a path for crossing an energy barrier of a free energy curved surface when sampling the structure of the cyclic peptide will be described in more detail with reference to FIG. 3.

In the example illustrated in FIG. 3, the magnitude of the free energy on the free energy curved surface is represented by contour lines. In FIG. 3, regions with the lowest free energy (which has a stable structure) are designated as regions L1 and L2, and a region with the highest free energy is designated as a region H with reference to the outermost contour line. Note that, in FIG. 3, the contour line of the region lower than the reference is represented by a solid line, and the contour line of the region higher than the reference is represented by a dotted line, where the outermost contour line is the reference.

Here, in FIG. 3, when sampling the structure, consider a case where two models having parameters determined on the basis of different initial structures are prepared, and the sampling is performed from a structure P located in the region L1 to be a stable structure to include the structure of the region L2 to be another stable structure.

As illustrated in FIG. 3, for example, in a model based on the initial structure having the parameters passing through a path a when performing the sampling by the extended ensemble method, there is the region H (energy barrier) with the highest free energy in the way from the region L1 to the region L2. Therefore, in the model based on the initial structure having the parameters passing through the path a, the region H is not able to be crossed, and the structure of the region L2 to be another stable structure is not able to be sampled.

Meanwhile, as in an example illustrated in FIG. 3, in a model based on the initial structure having the parameters passing through a path b, there is no region H (energy barrier) with the highest free energy on the path from the region L1 to the region L2, and the region L2 can be reached. Moreover, as illustrated in FIG. 3, in the model based on the initial structure having the parameters passing through the path b, the region L2 is reached via a wide range on the free energy curved surface. Therefore, the stable structure can be searched after structures in a wide structural topological range are sampled.

As described with reference to FIGs. 2 and 3, in the existing technique, when sampling a structure of a cyclic peptide, the structure of the cyclic peptide as the initial structure may affect the sampling result, and the sampled structure may be biased. In other words, in the existing technique, the sampling range for a structure depends on the initial structure, and the stable structure of the cyclic peptide may not be able to be sufficiently searched when sampling the structure of the cyclic peptide.

Moreover, as described above, in the cyclic peptide, the energy barrier separating the cis type and the trans type for the dihedral angle ω between a carbon atom and a nitrogen atom forming the peptide bond is high, and the energy barrier is less easily crossed even using the extended ensemble method. That is, when sampling the structure of the cyclic peptide, the sampling range may differ depending on whether the dihedral angle ω of the cyclic peptide used as the initial structure is the cis type or the trans type, and there is a case where the stable structure is not able to be sufficiently searched.

Here, the dihedral angle ω of the cyclic peptide is stable in the trans type for natural amino acid residues other than proline, but the cis type can also be taken for proline or modified amino acid residues such as N-methylation. For this reason, when modeling the structure of the cyclic peptide whose structure has not been determined by an experimental method (creating an initial structure), how to set the dihedral angle ω of proline or modified amino acid residues such as N-methylation becomes a problem.

Furthermore, the dihedral angle ω of proline or modified amino acid residues such as N-methylation in the cyclic peptide changes depending on a surrounding environment such as a target molecule or a solvent to be bound. For this reason, the dihedral angle ω at these amino acid residues is particularly difficult to specify by an experiment. Since the experiment is difficult in addition to the fact that there is often no index for setting the dihedral angle ω in the initial structure, it is especially needed to search for the stable structure by a computational method.

Therefore, the present inventor has studied a method of setting the initial structure enabling sampling of a similar structure to the case of using the initial structure based on an actual experiment result, by analyzing a correlation between the initial structure of the cyclic peptide used for sampling a structure and the sampled structure, and the like. Then, as a result of diligent studies, the present inventor has obtained the following findings regarding a method capable of appropriately sampling the structure of the cyclic peptide and appropriately searching for the stable structure of the cyclic peptide, and the like.

That is, the present inventor has found that the structure of the cyclic peptide can be appropriately sampled and the stable structure of the cyclic peptide can be appropriately searched by the following stable structure search method for a cyclic peptide, for example.

A stable structure search method for a cyclic peptide as an example of the technique disclosed in the present case is a stable structure search method for a cyclic peptide, including sampling a structure of the cyclic peptide, the stable structure search method including: causing a computer to perform the sampling, using a trans-type cyclic peptide structure in which all of dihedral angles ω between a carbon atom and a nitrogen atom forming a peptide bond in the cyclic peptide are from 150° to 210°, both inclusive, as an initial structure of a first molecular simulation.

Note that, in an example of the technique disclosed in the present case, a molecular simulation for sampling the structure of the cyclic peptide may be referred to as a "first molecular simulation".

Here, in an example of the technique disclosed in the present case, the trans-type cyclic peptide structure in which all the dihedral angles ω between a carbon atom and a nitrogen atom forming a peptide bond in the cyclic peptide are from 150° to 210°, both inclusive, is set as the initial structure of the first molecular simulation. In other words, in an example of the technique disclosed in the present case, the structure (trans-type cyclic peptide structure) in which all the dihedral angles ω of amino acid residues forming the cyclic peptide are from 150° to 210°, both inclusive, is set as the initial structure of the first molecular simulation. Note that, in an example of the technique disclosed in the present case, the structure in which all the dihedral angles ω between a carbon atom and a nitrogen atom forming a peptide bond are from 150° to 210°, both inclusive, in room temperature, for example, is favorably set as the trans-type cyclic peptide structure.

Furthermore, in an example of the technique disclosed in the present case, the trans-type cyclic peptide structure can be, for example, a structure in which all of the dihedral angles ω of the amino acid residues forming the cyclic peptide are of trans type. In an example of the technique disclosed in the present case, the case where the dihedral angle ω in the cyclic peptide is of the trans type can be a case where when the dihedral angle ω is expressed as an angle from 0° to 360°, the dihedral angle ω falls within a range from 150° to 210°, both inclusive, and is in the vicinity of 180° (substantially 180°). Furthermore, the case where the dihedral angle ω of the cyclic peptide is of the trans type can be a case where when the dihedral angle ω is expressed as an angle from 0° to 180° and from 0° to -180° (180° and -180° mean the same angle), the dihedral angle ω falls within a range from 150° to 180°, both inclusive, or from -180° to -150°, both inclusive.

Note that the case where the dihedral angle ω in the cyclic peptide is of the cis type can be a case where when the dihedral angle ω is expressed as an angle from 0° to 180° and from 0° to -180°, the dihedral angle ω falls within a range from -30° to 30°, both inclusive, and is in the vicinity of 0° (substantially 0°).

In an example of the technique disclosed in the present case, for example, when the dihedral angle ω of two amino acid residues is of the trans type, it means that side chains of these amino acid residues are located on different sides from each other across the double-bond character bond forming the dihedral angle ω. Meanwhile, in an example of the technique disclosed in the present case, for example, when the dihedral angle ω of two amino acid residues is of the cis type, it means that side chains of these amino acid residues are located mutually on the same side across the double-bond character bond forming the dihedral angle ω.

Then, in an example of the technique disclosed in the present case, the sampling is performed using the trans-type cyclic peptide structure in which all the dihedral angles ω in the cyclic peptide are from 150° to 210°, both inclusive, as the initial structure of the first molecular simulation. In other words, in an example of the technique disclosed in the present case, the structure of the cyclic peptide is sampled by executing the molecular simulation, setting the structure in which all the dihedral angles ω of the amino acid residues in the cyclic peptide are of the trans type, as the initial structure.

Here, in an example of the technique disclosed in the present case, since all of the dihedral angles ω of the cyclic peptide are of the trans type, the dihedral angles ω for the amino acid residues having the dihedral angle ω that can take the cis type are also made to the trans type, such as proline or modified amino acid residues such as N-methylation. As described above, in an example of the technique disclosed in the present case, the amino acid residues that can have a stable cis-type stable structure, such as proline or modified amino acid residues such as N-methylation, are unified to the trans type in the initial structure of the molecular simulation.

By doing so, as demonstrated in the examples to be described below, even for the cyclic peptides including proline having a cis-type stable structure, structures similar to those of the case using the initial structure based on an actual experiment result (the case where the stable structure is set as the initial structure) can be sampled. That is, in an example of the technique disclosed in the present case, the structure of the cyclic peptide can be appropriately sampled by setting the structure in which the dihedral angles ω of all the amino acid residues including the amino acid residues that can have the cis-type stable structure are of the trans type as the initial structure.

As described above, in an example of the technique disclosed in the present case, by using the initial structure in which the dihedral angles ω of all the amino acid residues are of the trans type, the energy barrier of the free energy curved surface can be appropriately crossed and sampling is performed. Therefore, the stable structure can be searched in a wide range of topology space.

As described above, in an example of the technique disclosed in the present case, the sampling is performed setting the trans-type cyclic peptide structure in which all the dihedral angles ω in the cyclic peptide are from 150° to 210°, both inclusive, as the initial structure of the first molecular simulation. By doing so, in an example of the technique disclosed in the present case, the structure of the cyclic peptide can be appropriately sampled, and the stable structure of the cyclic peptide can be appropriately searched.

Hereinafter, each process and the like in a stable structure search method for a cyclic peptide disclosed in the present case will be described in detail.

The stable structure search method for a cyclic peptide disclosed in the present case includes sampling a structure of the cyclic peptide (sampling process), for example, and further includes other steps as needed.

First, the stable structure search method for a cyclic peptide disclosed in the present case includes sampling the structure of the cyclic peptide. Then, in the stable structure search method for a cyclic peptide disclosed in the present case, a stable structure for the cyclic peptide is searched by performing sampling the structure of the cyclic peptide.

### <Cyclic Peptide>

Here, the cyclic peptide for searching for the stable structure is not particularly limited as long as the cyclic peptide is a cyclic molecule connected by a peptide bond using an amino acid residue as a monomer, and can be appropriately selected according to a purpose.

An amino acid that is a source of the amino acid residue may be a natural amino acid or an unnatural amino acid (modified amino acid or artificial amino acid). Examples of the natural amino acid include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, β-alanine, β-phenylalanine, and the like. Note that the number of amino acid residues in the cyclic peptide is not particularly limited and may be appropriately selected according to the purpose, and may be, for example, about 10 or more and 50 or less, or several hundreds.

Furthermore, an example of the modified amino acid includes an amino acid obtained by modifying (substituting) a part of the structure of the natural amino acid as described above, or the like. Specifically, as the modified amino acid, for example, an amino acid obtained by N-methylating a part of the structure of the natural amino acid or the like can be used.

Note that, regarding the cyclic peptide, in recent years, when cyclic peptides are used as drugs in the situations of drug discovery or the like, modified amino acids have been introduced to improve (cell) membrane permeability, physiological activity, and stability. Therefore, as described above, searching for the stable structure of the cyclic peptide containing the modified amino acid having the dihedral angle ω that can take the cis type is considered to be useful in the situations of drug discovery or the like.

In an example of the technique disclosed in the present case, as described above, the trans-type cyclic peptide structure in which all the dihedral angles ω between a carbon atom and a nitrogen atom forming a peptide bond in the cyclic peptide are from 150° to 210°, both inclusive, is set as the initial structure of the first molecular simulation. Then, in an example of the technique disclosed in the present case, the sampling of the structure of the cyclic peptide is performed by executing the first molecular simulation using the trans-type cyclic peptide structure as the initial structure.

### <Molecular Simulation>

Here, in an example of the technique disclosed in the present case, the molecular simulation for sampling the structure (first molecular simulation) is not particularly limited as long as the structure and movement of the molecules are obtained by numerical calculation by a computer, and can be appropriately selected according to a purpose. More specifically, as the molecular simulation for sampling the structure, for example, a molecular dynamics simulation can be favorably used.

The molecular dynamics (MD) simulation means, for example, simulating motion of particles (mass points) such as atoms by numerically solving a Newton's equation of motion. Furthermore, in the molecular dynamics simulation, there are an all-atom simulation in which each atom forming a molecule is explicitly handled, and a coarse-grained simulation in which a plurality of atoms is collectively coarse-grained and treated. In an example of the technique disclosed in the present case, for example, it is favorable to perform the all-atom simulation as the molecular dynamics simulation from the viewpoint of calculation accuracy.

The molecular dynamics simulation (molecular dynamics calculation) can be performed using, for example, a known molecular dynamics simulation program. Examples of the molecular dynamics simulation program include AMBER, CHARMm, GROMACS, GROMOS, NAMD, myPresto, MAPLECAFEE (registered trademark), and the like.

In the molecular dynamics simulation, for example, after creating the initial structure of the target molecule to be calculated, the size of a calculation system (calculation cell) is set, and a solvent molecule (for example, a water molecule) is placed around the target molecule. As a model of the water molecule, for example, a TIP3P model or the like can be used.

Then, in the molecular dynamics simulation, for example, Na⁺ ions, Cl⁻ ions, or the like are inserted into the calculation cell so that a total charge of atoms in the calculation cell becomes zero, and the force acting on each of the atoms is calculated under periodic boundary conditions. Then, in the molecular dynamics simulation, for example, how each atom included in the calculation system moves under the force is calculated on the basis of the Newton's equation of motion.

Here, in the molecular dynamics simulation, it is favorable to perform structural relaxation of the solvent molecule and size adjustment of the calculation system in order to carry out a more stable simulation. The structural relaxation of the solvent molecule is performed by, for example, molecular dynamics calculation (NVT calculation) with fixed particle count, volume, and temperature under the condition that the size of the calculation system is fixed and the positions of the atoms of the main chain of the target molecule are locked. Furthermore, the size adjustment of the calculation system can be performed by, for example, balancing the entire calculation system by molecular dynamics calculation (NPT calculation) with fixed particle count, pressure, and temperature after the structural relaxation of the solvent molecule.

Moreover, in the molecular dynamics simulation, for example, a more stable simulation can be continuously performed by executing the NVT calculation or the NPT calculation for the calculation cell for which the size has been adjusted as described above.

Furthermore, the molecular dynamics simulation is favorably performed at a set temperature from about 280 to 320 K (kelvin), both inclusive, for example.

Furthermore, in the molecular dynamics simulation, the force received by each atom included in the calculation system can be calculated on the basis of a molecular force field. Here, the molecular force field is, for example, a mathematical expression of the force that each atom existing in a molecule such as a peptide receives from another atom, as a potential function.

The molecular force field is not particularly limited and may be appropriately selected according to a purpose. For example, a created molecular force field may be used or an existing molecular force field may be used.

In the case of creating and using a molecular force field, for example, known molecular force field creation software can be used to create the molecular force field.

Regarding the existing molecular force field, examples of the molecular force field for a peptide include an Amber-based molecular force field, a CHARMm-based molecular force field, an OPLS-based molecular force field, and the like. Examples of the Amber-based molecular force field include Amber ff99SB-ILDN, Amber 12SB, and the like. An example of the CHARMm-based molecular force field includes CHARMm36 and the like. Furthermore, as the molecular force field, general AMBER force field (GAFF), which is a general-purpose molecular force field for organic compounds, can also be used.

Furthermore, in an example of the technique disclosed in the present case, the above-described molecular dynamics simulation using the extended ensemble method can be favorably used when sampling the structure of the cyclic peptide. Examples of the extended ensemble method include a multicanonical method, a replica exchange method, and the like.

In the multicanonical method, for example, the drop of the structure into the valley of a local solution can be suppressed by adding an artificial potential to implement a random walk in the energy space.

Furthermore, in the replica exchange method, a plurality of replicas (copies) of systems that do not interact with each other at different temperatures are prepared, simulations are independently performed for each replica, and two replicas with adjacent temperature values are exchanged under predetermined conditions. Therefore, in the replica exchange method, structures at various temperatures can be sampled, so that the drop of the structure into the valley of a local solution at a low temperature can be suppressed.

In this way, by sampling the structure of the cyclic peptide by the molecular dynamics simulation using various extended ensemble methods, the drop of the structure into the valley of a local solution can be suppressed, and the structure can be sampled in a wide range of the structural topological space.

### <Initial Structure>

Here, in an example of the technique disclosed in the present case, the method of obtaining the trans-type cyclic peptide structure in which all the dihedral angles ω between a carbon atom and a nitrogen atom forming a peptide bond in the cyclic peptide are from 150° to 210°, both inclusive, is not particularly limited, and can be appropriately selected according to a purpose. Examples of the method of obtaining the trans-type cyclic peptide structure include a method of manually setting the trans-type cyclic peptide structure, a method of extracting the trans-type cyclic peptide structure by performing the molecular simulation of slowly cooling the temperature from a high temperature state (having the cooling process), a method of extracting the trans-type cyclic peptide structure by performing the molecular simulation of reducing the value of the energy function regarding a solute and its surroundings, and the like.

In the method of manually setting the trans-type cyclic peptide structure, for example, a method of rotating and modifying an angle between a carbon atom and a nitrogen atom forming a peptide bond using molecular design software (molecular modeling software), a method of directly modifying atomic coordinates, or the like can be used. As the molecular design software, for example, "Discovery Studio" can be used.

As described above, in an example of the technique disclosed in the present case, it can be assumed that the trans-type cyclic peptide structure in which all the dihedral angles ω are manually set from 150° to 210°, both inclusive.

In the method of extracting the trans-type cyclic peptide structure by performing the molecular simulation (second molecular simulation having a cooling process) of slowly cooling the temperature from a high temperature state, for example, the molecular simulation of slowly cooling the temperature from a high-temperature state using an appropriately set parameter (molecular force field) is performed. Then, in the method of extracting the trans-type cyclic peptide structure by performing the molecular simulation of slowly cooling the temperature from a high temperature state, for example, the structure in which all the dihedral angles ω are of the trans type (from 150° to 210°, both inclusive) is appropriately extracted and selected from among the structures obtained in the molecular simulation.

More specifically, for example, the molecular dynamics simulation is performed so that a structure can freely appear under a high temperature condition of about 3000°C, using GROMACS that is molecular dynamics simulation software. Then, for example, the temperature in the molecular dynamics simulation is gradually lowered, the dihedral angle ω at this time is monitored (checked), and the structure in which all the dihedral angles ω become the trans type is selected.

As described above, in an example of the technique disclosed in the present case, it can be assumed that the trans-type cyclic peptide structure is extracted by performing the second molecular simulation having the cooling process as pretreatment of the first molecular simulation.

In the method of extracting the trans-type cyclic peptide structure by performing the simulation of reducing the value of the energy function (Hamiltonian) regarding a solute and its surroundings, the molecular simulation of reducing the magnitude of the energy function using the appropriately set parameter (molecular force field) is performed, for example. Then, in the method of extracting the trans-type cyclic peptide structure by performing the simulation of reducing the value (magnitude) of the energy function regarding a solute and its surroundings, for example, the structure in which all the dihedral angles ω are of the trans type (from 150° to 210°, both inclusive) is appropriately extracted and selected from among the structures obtained in the molecular simulation.

More specifically, for example, by introducing PLUMED that is plug-in software into GROMACS that is molecular dynamics simulation software to reduce the interaction between the solute (cyclic peptide) and water, and the molecular dynamics simulation is performed to enable the structure to freely appear. Then, for example, the dihedral angle ω in the molecular dynamics simulation is monitored (checked), and the structure in which all the dihedral angles ω become the trans type is selected.

As described above, in an example of the technique disclosed in the present case, it can be assumed that the trans-type cyclic peptide structure is extracted by performing a third molecular simulation of reducing the value of the energy function regarding a solute and its surroundings as pretreatment of the first molecular simulation.

Furthermore, in an example of the technique disclosed in the present case, for example, it is favorable to determine the parameters of the potential function including a charge (point charge) of each atom, van der Waals force, and the like, using the trans-type cyclic peptide structure in which all of the dihedral angles ω are of the trans type. In other words, in an example of the technique disclosed in the present case, for example, it is favorable to determine the parameters of the potential function used in the first molecular simulation on the basis of the trans-type cyclic peptide structure, and to create the model (trans-type cyclic peptide model) for the trans-type cyclic peptide.

That is, in an example of the technique disclosed in the present case, it is favorable to perform the sampling by the first molecular simulation, using the trans-type cyclic peptide model having the parameters determined on the basis of the trans-type cyclic peptide structure. By doing so, in an example of the technique disclosed in the present case, the structure can be sampled using more appropriate parameters, so that the sampling of an artificially erroneous structure can be avoided, and the stable structure of the cyclic peptide can be more appropriately searched.

Here, when determining the parameters of the potential function on the basis of the trans-type cyclic peptide structure, for example, an electronic state of the trans-type cyclic peptide structure is calculated using quantum chemical calculation software such as Gaussian or Gamess. Then, the parameters of the potential function are assigned using the above-described GAFF or the like, using antechamber (freeware of AmberTools distributed free of charge from Amber's development group) and the like.

Furthermore, when determining the parameters of the potential function on the basis of the trans-type cyclic peptide structure, the point charge is determined using a program called "respgen" of AmberTools for the charge of each atom. In this case, the determined point charge is a restrained electrostatic potential (RESP) charge.

### <Search for Stable Structure>

In an example of the technique disclosed in the present case, as described above, for example, the structure of the cyclic peptide is sampled by executing the first molecular simulation, setting the trans-type cyclic peptide structure as the initial structure, and the stable structure of the cyclic peptide is searched.

When searching for the stable structure of the cyclic peptide, for example, it is favorable to search for and specify the stable structure with low free energy by performing the free energy analysis for the sampled structure of the cyclic peptide. The method of the free energy analysis is not particularly limited and can be appropriately selected according to a purpose. For example, a method of calculating potential of mean force (PMF) using the sampled structure can be used. Furthermore, in the method of calculating the PMF, a structure having low PMF can be specified as the stable structure having low free energy.

### <Other Processes>

Other processes are not particularly limited and can be appropriately selected according to the purpose.

### (Cyclic Peptide Stable Structure Search Device)

A stable structure search device for a cyclic peptide disclosed in the present case is a stable structure search device for a cyclic peptide including: a sampling unit configured to perform sampling of a structure of the cyclic peptide, in which the sampling unit performs the sampling, using a trans-type cyclic peptide structure in which all of dihedral angles ω between a carbon atom and a nitrogen atom forming a peptide bond in the cyclic peptide are from 150° to 210°, both inclusive, as an initial structure of a first molecular simulation.

The stable structure search device for a cyclic peptide disclosed in the present case can be made to a device that executes the stable structure search method for a cyclic peptide disclosed in the present case, for example. Furthermore, a favorable mode in the stable structure search device for a cyclic peptide disclosed in the present case can be made similar to the favorable mode in the stable structure search method for a cyclic peptide disclosed in the present case, for example.

### (Stable Structure Search Program for Cyclic Peptide)

A stable structure search program for a cyclic peptide disclosed in the present case is a stable structure search program for a cyclic peptide, including sampling a structure of the cyclic peptide, the stable structure search program causing a computer to perform processing including: the sampling, using a trans-type cyclic peptide structure in which all of dihedral angles ω between a carbon atom and a nitrogen atom forming a peptide bond in the cyclic peptide are from 150° to 210°, both inclusive, as an initial structure of a first molecular simulation.

The stable structure search program for a cyclic peptide disclosed in the present case can be made to a program for causing a computer to execute the stable structure search method for a cyclic peptide disclosed in the present case, for example. Furthermore, a favorable mode in the stable structure search program for a cyclic peptide disclosed in the present case can be made similar to the favorable mode in the stable structure search method for a cyclic peptide disclosed in the present case, for example.

The stable structure search program for a cyclic peptide disclosed in the present case can be created using various known programming languages according to the configuration of a computer system to be used, the type and version of an operating system, and the like.

The stable structure search program for a cyclic peptide disclosed in the present case may be recorded in a recording medium such as a built-in hard disk or an externally attached hard disk, or may be recorded in a recording medium such as a CD-ROM, DVD-ROM, MO disk, or USB memory.

Moreover, in a case of recording the stable structure search program for a cyclic peptide disclosed in the present case in the above-described recording medium, the program can be directly used or can be installed into a hard disk and then used through a recording medium readout device included in the computer system, as needed. Furthermore, the stable structure search program for a cyclic peptide disclosed in the present case may be recorded in an external storage area (another computer or the like) accessible from the computer system through an information communication network. In this case, the stable structure search program for a cyclic peptide disclosed in the present case, which is recorded in the external storage area, can be used directly or can be installed in a hard disk and then used from the external storage area through the information communication network, as needed.

Note that the stable structure search program for a cyclic peptide disclosed in the present case may be divided for each of any pieces of processing and recorded in a plurality of recording media.

### (Computer-Readable Recording Medium)

A computer-readable recording medium disclosed in the present case records the stable structure search program for a cyclic peptide disclosed in the present case.

The computer-readable recording medium disclosed in the present case is not limited to any particular medium and can be appropriately selected according to the purpose. Examples of the computer-readable recording medium include a built-in hard disk, an externally attached hard disk, a CD-ROM, a DVD-ROM, an MO disk, a USB memory, and the like, for example.

Furthermore, the computer-readable recording medium disclosed in the present case may be a plurality of recording media in which the stable structure search program for a cyclic peptide disclosed in the present case is divided and recorded for each of any pieces of processing.

Hereinafter, an example of the technique disclosed in the present case will be described in more detail using configuration examples of the device, flowcharts, and the like.

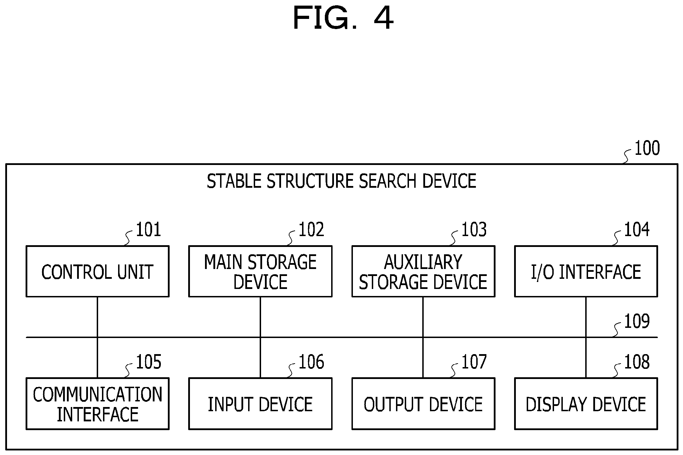

FIG. 4 illustrates a hardware configuration example of the stable structure search device for a cyclic peptide disclosed in the present case. Note that, hereinafter, the stable structure search device for a cyclic peptide disclosed in the present case may be simply referred to as a "stable structure search device".

In a stable structure search device 100, for example, a control unit 101, a main storage device 102, an auxiliary storage device 103, an I/O interface 104, a communication interface 105, an input device 106, an output device 107, and a display device 108 are connected to one another via a system bus 109.

The control unit 101 performs arithmetic operations (for example, four arithmetic operations, comparison operations, and arithmetic operations for the annealing method), hardware and software operation control, and the like. As the control unit 101, for example, a processor such as a central processing unit (CPU) can be adopted.

The control unit 101 implements various functions, for example, by executing the program (for example, the stable structure search program for a cyclic peptide disclosed in the present case or the like) read in the main storage device 102 or the like.

The processing performed by the sampling unit in the stable structure search device for a cyclic peptide disclosed in the present case can be performed by, for example, the control unit 101.

The main storage device 102 stores various programs and data or the like needed for executing various programs. As the main storage device 102, for example, a device having at least one of a read only memory (ROM) and a random access memory (RAM) can be used.

The ROM stores various programs, for example, a basic input/output system (BIOS) or the like. Furthermore, the ROM is not particularly limited and can be appropriately selected according to the purpose. For example, a mask ROM, a programmable ROM (PROM), or the like can be exemplified.

The RAM functions, for example, as a work range expanded when various programs stored in the ROM, the auxiliary storage device 103, or the like are executed by the control unit 101. The RAM is not particularly limited and can be appropriately selected according to the purpose. For example, a dynamic random access memory (DRAM), a static random access memory (SRAM), or the like can be exemplified.

The auxiliary storage device 103 is not particularly limited as long as the device can store various information and can be appropriately selected according to the purpose. For example, a solid state drive (SSD), a hard disk drive (HDD), or the like can be exemplified. Furthermore, the auxiliary storage device 103 may be a portable storage device such as a CD drive, a DVD drive, or a Blu-ray (registered trademark) disc (BD) drive.

Furthermore, the stable structure search program for a cyclic peptide disclosed in the present case is, for example, stored in the auxiliary storage device 103, loaded into the RAM (main memory) of the main storage device 102, and executed by the control unit 101.

The I/O interface 104 is an interface used to connect various external devices. The I/O interface 104 can input/output data to/from, for example, a compact disc ROM (CD-ROM), a digital versatile disk ROM (DVD-ROM), a magneto-optical disk (MO disk), a universal serial bus (USB) memory (USB flash drive), or the like.

The communication interface 105 is not particularly limited, and a known communication interface can be appropriately used. For example, a communication device using wireless or wired communication or the like can be exemplified.

The input device 106 is not particularly limited as long as the device can receive input of various requests and information with respect to the stable structure search device 100, a known device can be appropriately used. For example, a keyboard, a mouse, a touch panel, a microphone, or the like can be exemplified. Furthermore, in a case where the input device 106 is a touch panel (touch display), the input device 106 can also serve as the display device 108.

The output device 107 is not particularly limited, and a known device can be appropriately used. For example, a printer or the like can be exemplified.

The display device 108 is not particularly limited, and a known device can be appropriately used. For example, a liquid crystal display, an organic EL display, or the like can be exemplified.

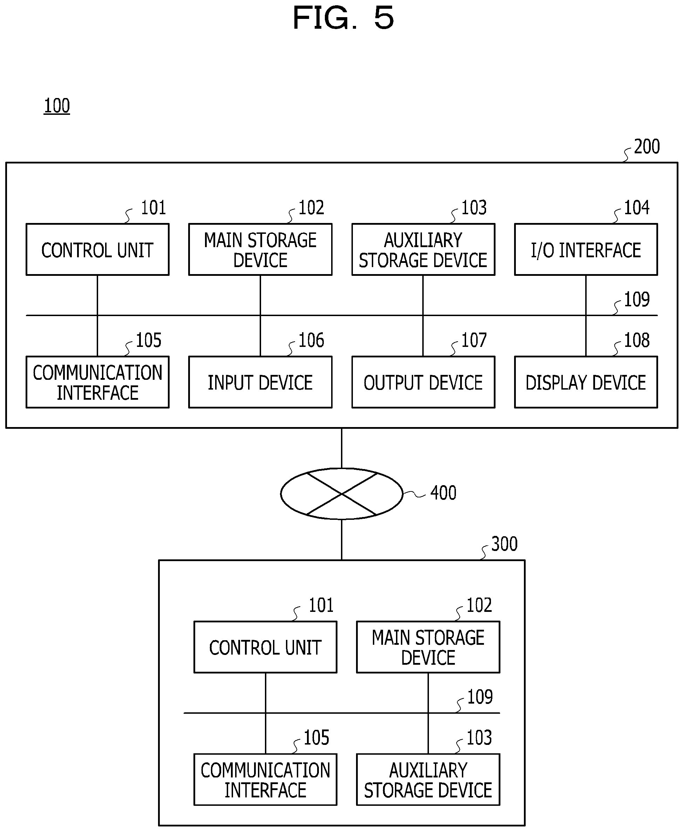

FIG. 5 illustrates another hardware configuration example of the stable structure search device for a cyclic peptide disclosed in the present case.

In the example illustrated in FIG. 5, the stable structure search device 100 is divided into a terminal device 200 and a server computer 300. The terminal device 200 performs processing such as setting the initial structure and parameters of the cyclic peptide used in the molecular simulation, and outputting a search result of the stable structure, for example. Meanwhile, the server computer 300 performs the molecular simulation for sampling the structure of the cyclic peptide, for example. Furthermore, in the example illustrated in FIG. 5, the terminal device 200 and the server computer 300 in the stable structure search device 100 are connected via a network 400.

In the example illustrated in FIG. 5, for example, as the terminal device 200, a normal personal computer can be used, and as the server computer 300, a computer cluster in which a plurality of computers is connected, a large-scale and high-performance computer such as a supercomputer can be used. Note that the server computer 300 may be a group of computers on the cloud.

Furthermore, as the network 400 connecting the terminal device 200 and the server computer 300, for example, a communication standard such as Secure Shell (SSH) can be used.

In the example illustrated in FIG. 5, for example, the terminal device 200 sets the initial structure and parameters of the cyclic peptide used in the molecular simulation. Then, a condition file needed for the molecular simulation created by the terminal device 200 is transmitted from the terminal device 200 to the server computer 300 via the network 400.

The server computer 300 then performs the molecular simulation for sampling the structure of the cyclic peptide. Then, data (trajectory file and the like) of the result of the molecular simulation is transmitted from the server computer 300 to the terminal device 200 via the network 400.

Next, the terminal device 200 searches for the stable structure of the cyclic peptide on the basis of the received data of the result of the molecular simulation, and outputs the result.

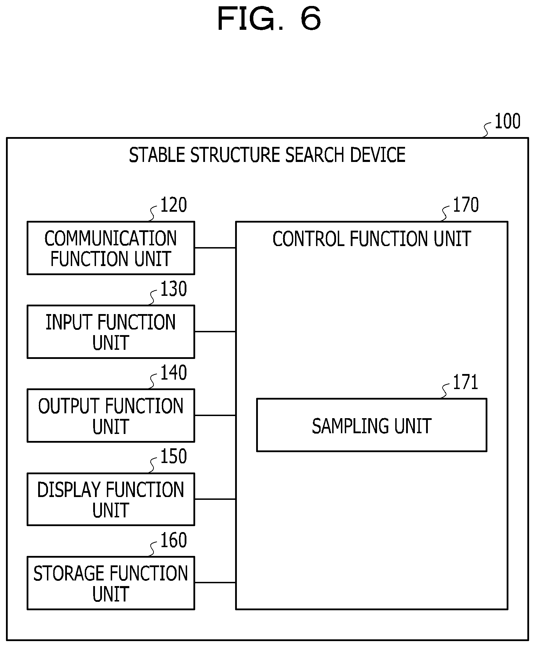

FIG. 6 illustrates a functional configuration example of the stable structure search device for a cyclic peptide disclosed in the present case.

As illustrated in FIG. 6, the stable structure search device 100 includes a communication function unit 120, an input function unit 130, an output function unit 140, a display function unit 150, a storage function unit 160, and a control function unit 170.

The communication function unit 120, for example, transmits and receives various data to and from an external device.

The input function unit 130 accepts, for example, various instructions for the stable structure search device 100.

The output function unit 140 prints and outputs, for example, the data of the searched stable structure of the cyclic peptide or the like.

The display function unit 150 displays, for example, the data of the searched stable structure of the cyclic peptide on a display or the like.

The storage function unit 160 stores, for example, various programs, the initial structure of the cyclic peptide, a parameter file (topology file) used for molecular dynamics simulation, data of the searched stable structure of the cyclic peptide, and the like.

The control function unit 170 includes a sampling unit 171. The control function unit 170 executes, for example, the various programs stored in the storage function unit 160 and controls the operation of the entire stable structure search device 100.

The sampling unit 171 performs sampling, using, for example, the trans-type cyclic peptide structure in which all the dihedral angles ω between a carbon atom and a nitrogen atom forming a peptide bond in the cyclic peptide are from 150° to 210°, both inclusive, as the initial structure of the molecular simulation.

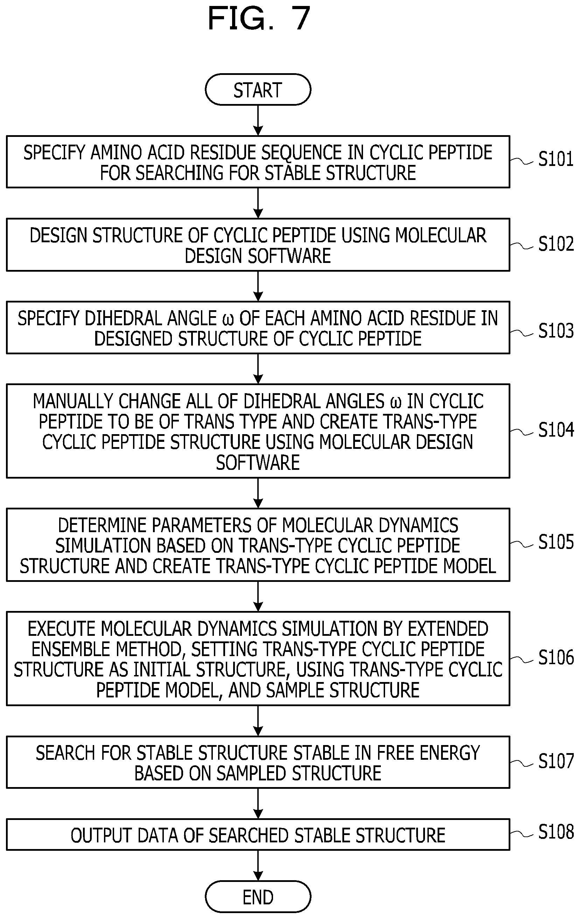

FIG. 7 illustrates a flowchart illustrating an example of a flow when searching for a stable structure of a cyclic peptide using the technique disclosed in the present case.

First, in an example of the technique disclosed in the present case, an amino acid residue sequence in the cyclic peptide for searching for the stable structure is specified (S101). More specifically, in S101, the type of the amino acid residue contained in the cyclic peptide for searching for the stable structure and the order of binding the amino acid residue in the cyclic peptide are specified.

Next, in an example of the technique disclosed in the present case, the structure of the cyclic peptide is designed using the molecular design software (S102). In other words, in S102, the structure of the cyclic peptide is created on the basis of information of the amino acid sequence of the cyclic peptide for searching for the stable structure, using the molecular design software.

Then, in an example of the technique disclosed in the present case, the dihedral angle ω of each amino acid residue in the designed structure of the cyclic peptide is specified (S103). More specifically, in S103, for example, the dihedral angle ω of each amino acid residue is calculated and specified on the basis of information of the dihedral angle between a carbon atom and a nitrogen atom forming a peptide bond of each amino acid residue in the designed structure of the cyclic peptide.

Next, in an example of the technique disclosed in the present case, the trans-type cyclic peptide structure is created by manually changing all of the dihedral angles ω in the cyclic peptide to be of trans type, using the molecular design software (S104). More specifically, in S104, for example, the trans-type cyclic peptide structure is created by manually changing the structure of the cyclic peptide such that all the dihedral angles ω in the cyclic peptide fall in the range from 150° to 210°, both inclusive, on the molecular design software.

Then, in an example of the technique disclosed in the present case, the sampling unit 171 determines parameters of the molecular dynamics simulation on the basis of the trans-type cyclic peptide structure, and creates the trans-type cyclic peptide model (S105). In other words, in S105, the sampling unit 171 creates the trans-type cyclic peptide model having the parameters determined in S104 on the basis of the trans-type cyclic peptide structure.

Next, in an example of the technique disclosed in the present case, the sampling unit 171 executes the molecular dynamics simulation by the extended ensemble method, using the trans-type cyclic peptide structure as the initial structure, using the trans-type cyclic peptide model (S106). More specifically, in S106, the sampling unit 171 samples the structure of the cyclic peptide by executing the molecular dynamics simulation by the extended ensemble method, setting the trans-type cyclic peptide structure as the initial structure, using the parameters determined in S105, for example.

Next, in an example of the technique disclosed in the present case, the sampling unit 171 searches for a stable structure that is stable in free energy on the basis of the sampled structure (S107). More specifically, in S107, the sampling unit 171 specifies, for example, the free energy profile of the cyclic peptide on the basis of the sampled structure, and specifies a structure having low free energy as the stable structure.

Next, in an example of the technique disclosed in the present case, the sampling unit 171 outputs data of the searched stable structure (S108). More specifically, in S108, the sampling unit 171 outputs structural data (data expressing a three-dimensional structure), free energy value data, and the like for the stable structure specified in S107.

Then, in an example of the technique disclosed in the present case, the processing is terminated when output of the data of the searched stable structure is completed.

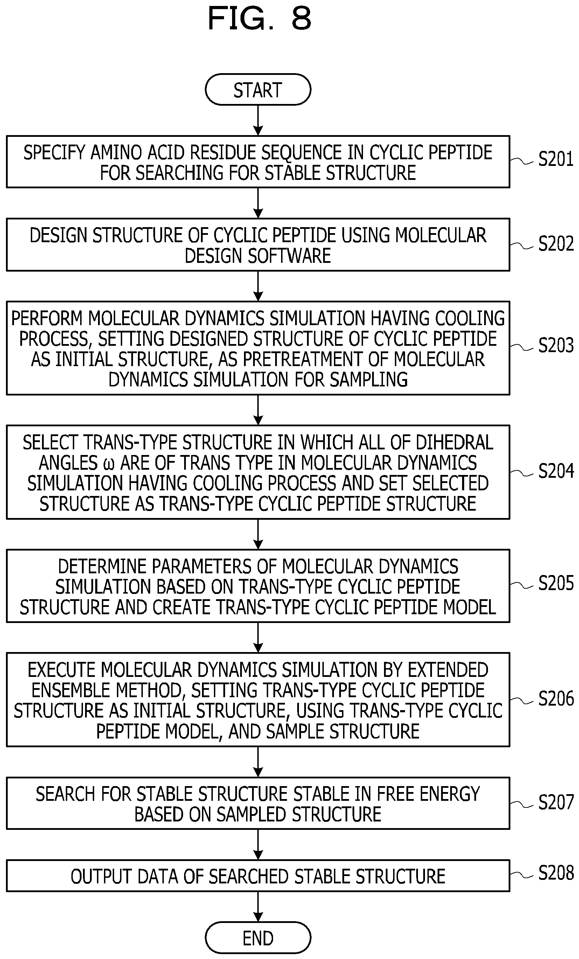

FIG. 8 illustrates a flowchart illustrating another example of the flow when searching for a stable structure of a cyclic peptide using an example of the technique disclosed in the present case. Note that, since S201, S202, and S205 to S208 in the flowchart illustrated in FIG. 8 can be respectively made similar to S101, S102, and S105 to S108 in the flowchart illustrated in FIG. 7, description thereof is be omitted.

In S203, in an example of the technique disclosed in the present case, the molecular dynamics simulation (under the condition of slow cooling from a high-temperature state) having a cooling process, setting the set structure of the cyclic peptide as the initial structure, is performed as pretreatment of the molecular dynamics simulation for sampling (S203). More specifically, in S203, for example, the molecular dynamics simulation is performed under the condition that the temperature is gradually lowered after allowing the structure to freely appear at a high temperature of about 3000°C.

Next, in an example of the technique disclosed in the present case, in the molecular dynamics simulation having the cooling process, a structure in which all the dihedral angles ω are of trans type is selected and specified as the trans-type cyclic peptide structure (S204). More specifically, in S204, for example, the dihedral angle ω during execution of the molecular dynamics simulation is monitored (checked), and the structure in which all the dihedral angle ω become the trans type is selected, and specifies the selected structure as the trans-type cyclic peptide structure.

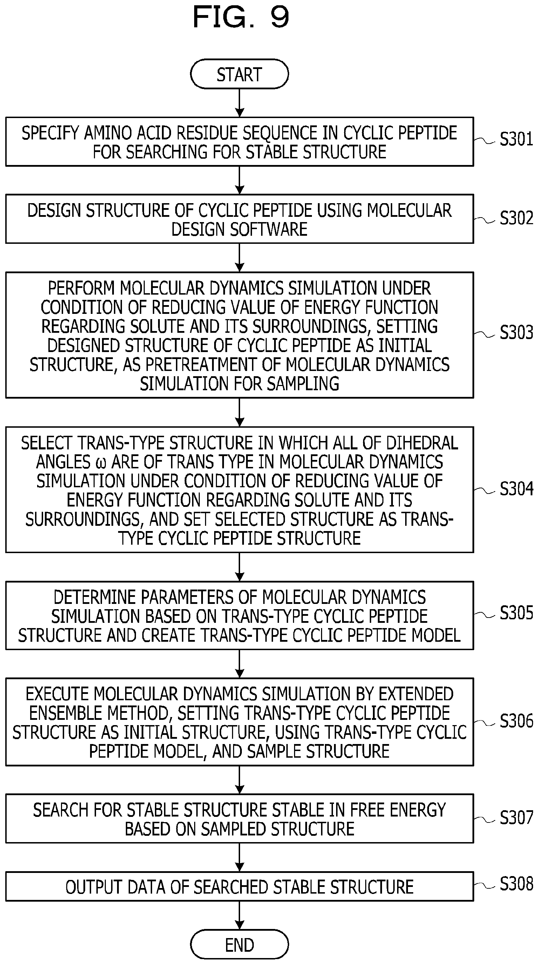

FIG. 9 illustrates a flowchart illustrating another example of the flow when searching for a stable structure of a cyclic peptide using an example of the technique disclosed in the present case. Note that, since S301, S302, and S305 to S308 in the flowchart illustrated in FIG. 9 can be respectively made similar to S101, S102, and S105 to S108 in the flowchart illustrated in FIG. 7, description thereof is be omitted.

In S303, in an example of the technique disclosed in the present case, the molecular dynamics simulation under the condition of reducing the value of the energy function regarding a solute and its surroundings, setting the designed structure of the cyclic peptide as the initial structure, is performed as pretreatment of the molecular dynamics simulation for sampling (S303). More specifically, in S303, for example, the interaction between the solute (cyclic peptide) and water is reduced, and the molecular dynamics simulation is performed so that the structure freely appears.

Next, in an example of the technique disclosed in the present case, in the molecular dynamics simulation under the condition of reducing the value of the energy function regarding a solute and its surroundings, a structure in which all the dihedral angles ω are of trans type is selected and specified as the trans-type cyclic peptide structure (S304). More specifically, in S304, for example, the dihedral angle ω during execution of the molecular dynamics simulation is monitored (checked), and the structure in which all the dihedral angle ω become the trans type is selected, and specifies the selected structure as the trans-type cyclic peptide structure.

Furthermore, in FIGs. 7 to 9, the flow of the processing in an example of the technique disclosed in the present case has been described according to a specific order. However, in the technology disclosed in the present case, it is possible to appropriately switch an order of each steps in a technically possible range. Furthermore, in the technology disclosed in the present case, a plurality of steps may be collectively performed in a technically possible range.

### [Example]

An example of the technology disclosed in the present case will be described. However, the technology disclosed in the present case is not limited to the example.

In the present example, for a cyclic peptide formed using eight amino acid residues and having a three-dimensional structure specified by nuclear magnetic resonance (NMR), the correlation between the initial structure of the cyclic peptide and the sampled structure has been analyzed. More specifically, in the present example, an analysis has been performed for the cyclic peptide (PDB ID: 6AXI) having the amino acid residue sequence of "aspartic acid (D)-leucine (L)-phenylalanine (F)-valine (V)-proline (P)-proline (P)-isoleucine (I)-aspartic acid (D)".

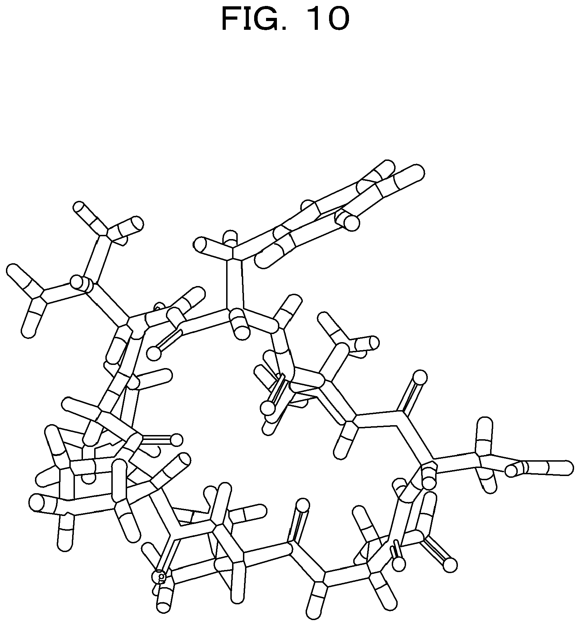

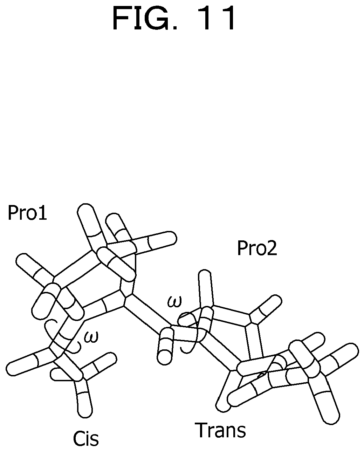

FIG. 10 illustrates an example of the structure of the cyclic peptide (PDB ID: 6AXI) targeted in the present example. Furthermore, FIG. 11 illustrates an example of the stable structure specified in an experiment at a site where two prolines are consecutively bound in the cyclic peptide (PDB ID: 6AXI) targeted in the present example.

As illustrated in FIG. 11, in the cyclic peptide targeted in the present example, the structure in which the first proline (Pro1) becomes the cis type (Cis) and the second proline (Pro2) becomes the trans type (Trans) at the site where the two prolines are consecutively bound is the stable structure, which has been specified in the experiment.

In the present example, for the above cyclic peptide, the structure has been sampled by executing the molecular dynamics simulation, appropriately setting the dihedral angle ω of the initial structure of the above cyclic peptide, using a structure search device having the hardware configuration as illustrated in FIG. 5.

More specifically, in the present example, four types of initial structures have been respectively created by setting the dihedral angle ω at the site where the two prolines of the above cyclic peptide are consecutive to "cis type-trans type (corresponding to the stable structure specified in the experiment)", "trans type-trans type", "trans type-cis type", and "cis type-cis type". Furthermore, in the present example, all the dihedral angles ω at the amino acid residues other than proline in the initial structure of the cyclic peptide have been set to the trans type.

Note that, in the present example, the above-described initial structure of the cyclic peptide has been created by manually changing the dihedral angle ω of each amino acid residue, using "Discovery Studio" as the molecular design software, using the structure specified in an experiment.

Then, in the present example, the parameters needed for the molecular dynamics simulation have been determined using these four types of initial structures (models based on the initial structures have been created).

More specifically, in the present example, when determining the parameters of the potential functions for the created four types of initial structures, the electronic state of each initial structure has been calculated using quantum chemical calculation software. Then, in the present example, the parameters of the potential function have been assigned using the antechamber.

Furthermore, when determining the parameters of the potential functions for the created four types of initial structures, the point charge (RESP charge) has been determined using "respgen" of AmberTools for the charge of each atom and assigned as a parameter.

Next, in the present example, the structure has been sampled by executing the molecular dynamics simulation (an example of the first molecular simulation) using the extended ensemble method, for each of the created four types of initial structures, using the determined parameters. Note that, in the present example, the all-atom simulation has been performed as the molecular dynamics simulation.

Furthermore, in the present example, in the molecular dynamics simulation, a calculation system in which water molecules (TIP3P) are arranged around the cyclic peptide has been prepared, and the energy minimization calculation has been executed using the molecular dynamics simulation software GROMACS. Then, in the present example, the NVT calculation (the simulation under the conditions of fixed particle count, volume, and temperature) after setting the temperature to 298 K, using the molecular dynamics simulation software GROMACS. Then, in the present example, the structure of the cyclic peptide has been sampled by performing the NPT calculation (the simulation under the conditions of fixed particle count, pressure, and temperature) by applying the extended ensemble method.

Then, in the present example, the frequency distribution of the dihedral angle ω of the first proline (Pro1) at the site where two prolines are consecutively bound has been analyzed on the basis of the data obtained by sampling the structure of the cyclic peptide. Note that, in the analysis of the frequency distribution of the dihedral angle ω of the first proline (Pro1), the frequency distribution obtained using a PLUMED command driver has been drawn using Kaleidagraph (commercial software).

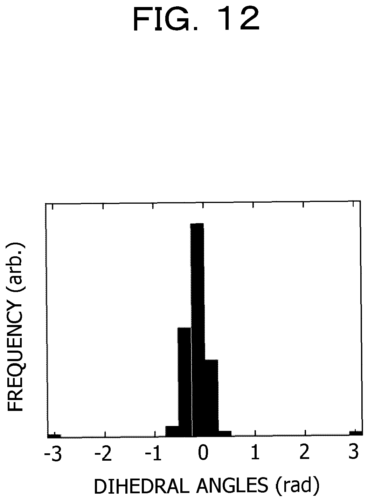

FIG. 12 illustrates an example of the frequency distribution of the dihedral angle ω of the first proline (Pro1) in the case of selecting, as an initial structure, the structure in which the first proline (Pro1) is the cis type and the second proline (Pro2) is the trans type at the site where the two prolines are consecutively bound in the cyclic peptide targeted in the present example. In FIG. 12, the vertical axis represents the frequency distribution (Frequency) of the dihedral angle ω of the first proline (Pro1), and the horizontal axis represents the values (Dihedral angles) of the dihedral angles ω of the first proline (Pro1). Furthermore, the unit of the horizontal axis in FIG. 12 is radian (rad). For example, the vicinity of "3" corresponds to the vicinity of "180°" and the vicinity of "-3" corresponds to the vicinity of "-180°".

As illustrated in FIG. 12, the dihedral angle ω of the first proline (Pro1) became the cis type in the vicinity of 0° most frequently in the case of setting the structure in which the first proline (Pro1) is the cis type and the second proline (Pro2) is the trans type (corresponding to the stable structure specified in an experiment) as the initial structure. In this way, in the case of setting the structure (cis type-trans type) corresponding to the stable structure specified in the experiment as the initial structure, the stable structure specified in the experiment has been maintained even in the sampling of the structure.

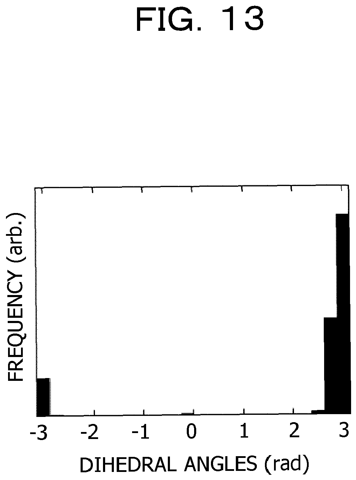

FIG. 13 illustrates an example of the frequency distribution of the dihedral angle ω of the first proline (Pro1) in the case of selecting, as the initial structure, the structure in which the first proline (Pro1) is the cis type and the second proline (Pro2) is also the cis type at the site where the two prolines are consecutively bound in the cyclic peptide targeted in the present example.

As illustrated in FIG. 13, the dihedral angle ω of the first proline (Pro1) became the trans type in the vicinity of 180° (3rad) most frequently in the case of setting the structure in which the first proline (Pro1) is the cis type and the second proline (Pro2) is also the cis type as the initial structure. As described above, in the case of setting the structure in which the dihedral angle ω at the site where two cyclic peptide prolines are consecutively bound is the "cis type-cis-type" as the initial structure, a structure different from the stable structure specified in the experiment has been sampled, and appropriate sampling has not been able to be performed.

Furthermore, the dihedral angle ω of the first proline (Pro1) became the trans type in the vicinity of 180° (3rad) most frequently in the case of setting the structure in which the first proline (Pro1) is the trans type and the second proline (Pro2) is the cis type as the initial structure. As described above, in the case of setting the structure in which the dihedral angle ω at the site where two cyclic peptide prolines are consecutively bound is the "trans type-cis type" as the initial structure, a structure different from the stable structure specified in the experiment has been sampled, and appropriate sampling has not been able to be performed.

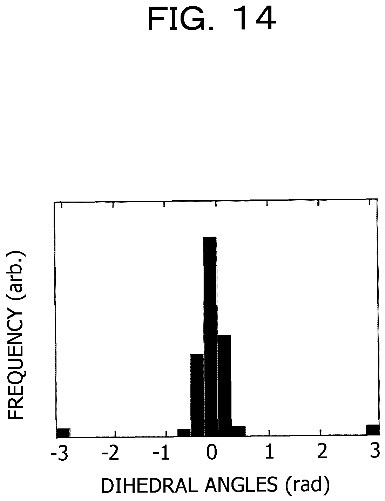

FIG. 14 illustrates an example of the frequency distribution of the dihedral angle ω of the first proline (Pro1) in the case of selecting, as the initial structure, the structure in which the first proline (Pro1) is the trans type and the second proline (Pro2) is also the trans type at the site where the two prolines are consecutively bound in the cyclic peptide targeted in the present example.

As illustrated in FIG. 14, the dihedral angle ω of the first proline (Pro1) became the cis type in the vicinity of 0° most frequently in the case of setting the structure in which the first proline (Pro1) is the trans type and the second proline (Pro2) is the trans type (the trans-type cyclic peptide structure in which all the dihedral angles ω are of the trans type) as the initial structure. As described above, in the case of setting the structure in which the dihedral angle ω at the site where two cyclic peptide prolines are consecutively bound is the "trans type-trans type" as the initial structure, a structure similar the stable structure specified in the experiment has been able to be sampled.

That is, in the present example, in the case of setting the dihedral angle ω at the site where two prolines of the cyclic peptide are consecutive to the "trans type-trans type", the frequency distribution similar to that of the case of setting the structure specified in the experiment as the initial structure has been able to be obtained.

That is, it has been confirmed from the result of the present example that a structure similar to that of the case of using the initial structure based on the actual experiment result can be sampled by setting the cyclic peptide containing proline having a cis-type stable structure to have the trans-type cyclic peptide structure as the initial structure. In other words, it has been confirmed from the result of the present example that the sampling by the molecular simulation setting the trans-type cyclic peptide structure in which all the dihedral angles ω are of the trans type as the initial structure can reproduce the result of the sampling by the molecular simulation using the initial structure based on the actual experiment result.

From the above results, it has been confirmed that the structure of the cyclic peptide can be appropriately sampled by setting the structure in which the dihedral angles ω of all the amino acid residues including the amino acid residues that can have the cis-type stable structure are of the trans type as the initial structure. Therefore, it has been confirmed that the stable structure of the cyclic peptide can be appropriately searched by performing sampling setting the trans-type cyclic peptide structure in which all the dihedral angles ω in the cyclic peptide are of the trans type as the initial structure of the molecular simulation.

## Claims

1. A stable structure search method for a computer to execute a process comprising:
determining an initial structure of a trans-type cyclic peptide so that all of dihedral angles between a carbon atom and a nitrogen atom that form a peptide bond in the trans-type cyclic peptide are from 150° to 210°; and
performing a first molecular simulation of a structure of the trans-type cyclic peptide by using the initial structure.

2. The stable structure search method according to claim 1, wherein the process further comprising
performing a second molecular simulation of the structure of the trans-type cyclic peptide with cooling the trans-type cyclic peptide before the first molecular simulation.

3. The stable structure search method according to claim 1, wherein the process further comprising
performing a third molecular simulation of the structure of the trans-type cyclic peptide with reducing a value of an energy function that regards a solute and a surrounding of the solute before the first molecular simulation.

4. The stable structure search method according to any one of claims 1 to 3, wherein the process further comprising
using a trans-type cyclic peptide model that has a parameter determined based on the initial structure in the first molecular simulation.

5. A stable structure search program that causes at least one computer to execute a process, the process comprising:
determining an initial structure of a trans-type cyclic peptide so that all of dihedral angles between a carbon atom and a nitrogen atom that form a peptide bond in the trans-type cyclic peptide are from 150° to 210°; and
performing a first molecular simulation of a structure of the trans-type cyclic peptide by using the initial structure.

6. The stable structure search program according to claim 5, wherein the process further comprising
performing a second molecular simulation of the structure of the trans-type cyclic peptide with cooling the trans-type cyclic peptide before the first molecular simulation.

7. The stable structure search program according to claim 5, wherein the process further comprising
performing a third molecular simulation of the structure of the trans-type cyclic peptide with reducing a value of an energy function that regards a solute and a surrounding of the solute before the first molecular simulation.

8. The stable structure search program according to any one of claims 5 to 7, wherein the process further comprising
using a trans-type cyclic peptide model that has a parameter determined based on the initial structure in the first molecular simulation.

9. A stable structure search device comprising:
a sampling unit that
determines an initial structure of a trans-type cyclic peptide so that all of dihedral angles between a carbon atom and a nitrogen atom that form a peptide bond in the trans-type cyclic peptide are from 150° to 210°, and
performs a first molecular simulation of a structure of the trans-type cyclic peptide by using the initial structure.

10. The stable structure search device according to claim 9, wherein the sampling unit is further configured to
perform a second molecular simulation of the structure of the trans-type cyclic peptide with cooling the trans-type cyclic peptide before the first molecular simulation.

11. The stable structure search device according to claim 9, wherein the sampling unit is further configured to
perform a third molecular simulation of the structure of the trans-type cyclic peptide with reducing a value of an energy function that regards a solute and a surrounding of the solute before the first molecular simulation.

12. The stable structure search device according to any one of claims 9 to 11, wherein the sampling unit is further configured to
use a trans-type cyclic peptide model that has a parameter determined based on the initial structure in the first molecular simulation.
